(19) Europäisches Patentamt
European Patent Office
Office européen des brevets

(11) Veröffentlichungsnummer: **0 156 198**
**B1**

# (12) EUROPÄISCHE PATENTSCHRIFT

(45) Veröffentlichungstag der Patentschrift: **20.04.88**

(51) Int. Cl.⁴: **C 07 D 271/08** // A01N47/36

(21) Anmeldenummer: **85102444.8**

(22) Anmeldetag: **05.03.85**

## (54) Verfahren zur Herstellung von Isocyanaten.

(30) Priorität: **17.03.84 DE 3409887**

(43) Veröffentlichungstag der Anmeldung: **02.10.85 Patentblatt 85/40**

(45) Bekanntmachung des Hinweises auf die Patenterteilung: **20.04.88 Patentblatt 88/16**

(84) Benannte Vertragsstaaten: **AT BE CH DE FR GB IT LI NL**

(56) Entgegenhaltungen:
**FR - A - 1 071 629**

**HOUBEN-WEYL: "Methoden der organischen Chemie", Erweiterungs- und Folgebände zur vierten Auflage, Band E4, 1983, Seiten 750-751, Georg Thieme Verlag, Stuttgart, DE;**
**ANGEW. CHEM. 80 362-363 (1968)**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Sirrenberg, Wilhelm, Dr., Wuppertaler Strasse 21, D-4322 Sprockhövel (DE)**
Erfinder: **Heitkämper, Peter, Dr., Fliederweg 14, D-4047 Dormagen 11 (DE)**
Erfinder: **König, Klaus, Dr., Heymannstrasse 50, D-5090 Leverkusen 1 (DE)**

**EP 0 156 198 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von neuen 3-Isocyanato-1,2,5-oxadiazolen durch die Umsetzung von 3-Amino-1,2,5-oxadiazolen oder entsprechenden N'-substituierten 3-Ureido-1,2,5-oxadiazolen mit Phosgen bei Temperaturen zwischen 150 und 210 °C. Die 3-Isocyanato-1,2,5-oxadiazole können als wertvolle Zwischenprodukte bei organischchemischen Synthesen auf vielen Gebieten, z. B. zur Herstellung von Pflanzenschutzmitteln, Pharmazeutika, Farbstoffen usw. eingesetzt werden.

Die Herstellung zahlreicher Isocyanate aus den entsprechenden Aminen durch die Umsetzung mit Phosgen ist bereits bekannt. Weiterhin ist es bekannt, dass N-Heterocyclen mit einer Isocyanat-Gruppe in α-Stellung instabil sind und zu spontanen Cyclisierungsreaktionen neigen [vgl. Angew. Chem. 80, 362–363 (1968)].

Es wurde gefunden, dass man die neuen 3-Isocyanato-1,2,5-oxadiazole der allgemeinen Formel I

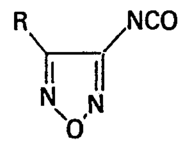

(I)

in welcher

R für einen gegebenenfalls substituierten aromatischen Rest steht, welcher unter den erfindungsgemässen Verfahrensbedingungen mit Phosgen nicht reagiert,

erhält, wenn man Verbindungen der allgemeinen Formel II

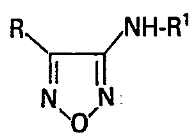

(II)

in welcher

R die oben angegebene Bedeutung hat und

$R^1$ für Wasserstoff oder für die Gruppen der allgemeinen Formeln III oder IV

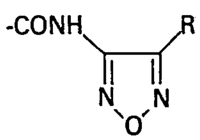

(III)

$-CONHR^2$ (IV)

steht, in welchen

R die oben angegebene Bedeutung hat und

$R^2$ einen gegebenenfalls substituierten aliphatischen oder aromatischen Rest bedeutet,

mit Phosgen bei Temperaturen zwischen 150 und 220 °C in Gegenwart eines organischen Verdünnungsmittels umsetzt und das erhaltene Isocyanat der allgemeinen Formel I nach den üblichen Methoden isoliert und gegebenenfalls reinigt.

Im Hinblick auf den vorbekannten Stand der Technik war es überraschend und für den Fachmann nicht vorhersehbar, dass nach dem erfindungsgemässen Verfahren die Verbindungen der allgemeinen Formel I in guter Ausbeute und hoher Reinheit auf einfache Weise erhalten werden können. Bei Kenntnis des Standes der Technik wäre eigentlich zu erwarten gewesen, dass sich bei den erfindungsgemäss anzuwendenden Reaktionsbedingungen entstandene Isocyanate noch im Verlauf der Reaktion in erheblichem Masse zersetzten bzw. zu unerwünschten Produkten weiterreagieren würden [vgl. Angew. Chem. 80, 362–363 (1968)]. Unter schonenderen Bedingungen (wie niedrigere Temperaturen, Basenzusatz) werden die gewünschten Produkte nur in unzureichendem Masse erhalten.

Die gegebenenfalls substituierten aromatischen Reste R und $R^2$ der obigen Formeln enthalten vorzugsweise 6 oder 10 Kohlenstoffatome im Arylteil. Beispielhaft seien gegebenenfalls substituiertes Phenyl oder Naphthyl, vorzugsweise Phenyl, genannt.

Der gegebenenfalls substituierte aliphatische Rest $R^2$ steht vorzugsweise für gegebenenfalls substituiertes geradkettiges oder verzweigtes Alkyl mit 1 bis 12, vorzugsweise 1 bis 8 und insbesondere 1 bis 6 Kohlenstoffatomen sowie für gegebenenfalls substituiertes Cycloalkyl mit vorzugsweise 1 bis 8, insbesondere 5 oder 6 Kohlenstoffatomen. Beispielhaft seien Methyl, Ethyl, n- und i-Propyl, n-, i-, s- und t-Butyl sowie Cyclopentyl und Cyclohexyl genannt.

Die aromatischen Reste R und $R^2$ sowie der aliphatische Rest $R^2$ können einen oder mehrere, vorzugsweise 1 bis 5, insbesondere 1 bis 3 und besonders bevorzugt 1 oder 2 gleiche oder verschiedene Substituenten tragen. Als Substituenten kommen alle Substituenten in Frage, welche unter den erfindungsgemässen Reaktionsbedingungen mit Phosgen nicht reagieren.

Beispielhaft seien aufgeführt:

Halogen, vorzugsweise Fluor, Chlor, Brom und Jod, insbesondere Fluor, Chlor und Brom; Alkyl mit vorzugsweise 1 bis 6, insbesondere 1 bis 4 Kohlenstoffatomen, wie Methyl, Ethyl, n- und i-Propyl, n-, i-, sec-, und tert-Butyl; Alkoxy mit vorzugsweise 1 bis 6, insbesondere 1 bis 4 Kohlenstoffatomen, wie Methoxy, Ethoxy, n- und i-Propoxy, n-, i-, sec- und tert-Butoxy; Alkylthio mit vorzugsweise 1 bis 6, insbesondere 1 bis 4 Kohlenstoffatomen, wie Methylthio, Ethylthio, n- und i-Propylthio, n-, i-, sec- und tert-Butylthio; $C_1$–$C_4$-Alkoxy-$C_1$–$C_4$-Alkylthio; Cyano; Nitro; Halogenalkyl; Halogenalkoxy und Halogenalkylthio mit vorzugsweise jeweils 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor und/oder Brom, insbesondere Fluor und/oder Chlor stehen, wie Trifluormethyl, Trifluormethoxy, Trifluormethylthio, Chlortrifluorethoxy, Difluortrichlorethoxy, Dichlordifluorethoxy, Hexafluorn-propoxy, Chlordifluormethoxy und Chlordifluormethylthio; gegebenenfalls halogensubstituiertes $C_1$–$C_4$-Alkylcarbonyl, $C_1$–$C_4$-Alkoxycarbonyl, $C_1$–$C_4$-Alkoxycarbonyl-$C_1$–$C_2$-Alkyl und $C_1$–$C_4$-Alkoxycarbonyl-$C_1$–$C_4$-Alkylthio; gegebenenfalls durch

Fluor, Chlor und/oder Methyl substituiertes $C_1$–$C_4$-Alkylen, welches über zwei Sauerstoffatome an den Phenylring gebunden ist; Phenoxy und Phenylthio, wobei Phenoxy und Phenylthio ihrerseits wie die aromatischen Reste R und $R^2$ substituiert sein können.

Bevorzugt steht

R für Phenyl, welches durch Halogen, Nitro, Cyano, $C_1$–$C_4$-Alkyl, $C_1$–$C_4$-Alkoxy, $C_1$–$C_4$-Alkylthio, Halogen-$C_1$–$C_4$-Alkyl, Halogen-$C_1$–$C_4$-Alkoxy, Halogen-$C_1$–$C_4$-Alkylthio, Halogen-$C_1$–$C_4$-Alkylcarbonyl, $C_1$–$C_4$-Alkylcarbonyl, Halogen-$C_1$–$C_4$-Alkoxycarbonyl, $C_1$–$C_4$-Alkoxycarbonyl, $C_1$–$C_4$-Alkoxycarbonyl-$C_1$–$C_2$-Alkyl und/oder $C_1$–$C_4$-Alkoxycarbonyl-$C_1$–$C_4$-Alkylthio substituiert sein kann.

Besonders bevorzugt steht

R für Phenyl, welches substituiert sein kann durch Fluor, Chlor, Brom, Cyano, Nitro, Methyl, Ethyl, Methoxy, Ethoxy, Methylthio, Trifluormethyl, Trifluormethoxy, Trifluormethylthio, Methylcarbonyl, Methoxycarbonyl, Ethoxycarbonyl, n-Butoxycarbonyl, tert.-Butoxycarbonyl, Methoxycarbonylmethyl, Ethoxycarbonylmethyl, Methoxycarbonylmethylthio und/oder Ethoxycarbonylmethylthio.

Ganz besonders bevorzugt steht R für Phenyl, welches durch Fluor, Chlor und/oder Brom substituiert sein kann.

Bevorzugt steht $R^1$ für Wasserstoff, für die Gruppe der allgemeinen Formel III, in welcher R die oben als bevorzugte angegebene Bedeutung hat oder für die Gruppe der allgemeinen Formel IV, in welcher $R^2$ $C_1$–$C_6$-Alkyl oder Phenyl bedeutet.

Besonders bevorzugt steht $R^1$ für Wasserstoff, für die Gruppe der allgemeinen Formel III, in welcher R die oben als besonders bevorzugt angegebene Bedeutung hat oder für die Gruppe der allgemeinen Formel IV, in welcher $R^2$ $C_1$–$C_4$-Alkyl oder Phenyl bedeutet.

Halogen bedeutet in den vorstehenden Definitionen, wo nichts anderes angegeben wird, Fluor, Chlor, Brom oder Jod, vorzugsweise Fluor, Chlor und Brom, insbesondere Fluor oder Chlor.

Verwendet man 3-Amino-4-phenyl-1,2,5-oxadiazol als Ausgangsstoff (Formel I, R = Phenyl, $R^1$ = H), so kann der Reaktionsablauf durch das folgende Formelschema wiedergegeben werden:

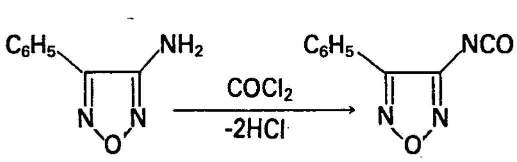


Verwendet man N-n-Butyl-N'-(4-phenyl-1,2,5-oxadiazol-3-yl)-harnstoff (Formel I, R = Phenyl, $R^1$ = Gruppe der Formel IV mit $R^2$ = n-Butyl) als Ausgangsstoff, so kann der Reaktionsablauf durch das folgende Formelschema wiedergegeben werden:

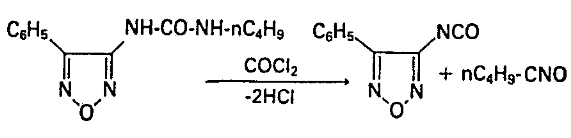


Verwendet man N,N'-Bis-(4-phenyl-1,2,5-oxadiazol-3-yl)-harnstoff (Formel I, $R^1$ = Gruppe der Formel III mit R = Phenyl) als Ausgangsstoff, so kann der Reaktionsablauf durch das folgende Formelschema wiedergegeben werden:

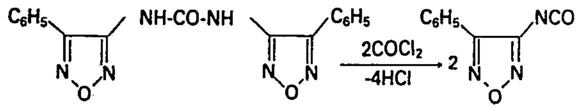


Die als Ausgangsstoffe zu verwendenden 3-Amino-1,2,5-oxadiazole der Formel II ($R^1$ = H) sind bekannt und/oder lassen sich nach literaturbekannten Verfahren und Methoden herstellen [vgl. J. Prakt. Chem. 315, 4, Seite 791–795 (1973)].

Die als Ausgangsstoffe zu verwendenden Oxadiazole der Formel (II) ($R^1$ = Gruppe der Formel III) sind teilweise bekannt und/oder nach üblichen Methoden erhältlich. Sie können beispielsweise aus den Verbindungen der Formel II ($R^1$ = H) durch die Umsetzung mit Phosgen bei Temperaturen von 50 bis 130 °C in einem inerten organischen Verdünnungsmittel, z.B. Chlorbenzol, leicht hergestellt werden.

Die als Ausgangsstoffe zu verwendenden Oxadiazole der Formel II ($R^1$ = Gruppe der Formel IV) sind bekannt oder können nach allgemein üblichen Verfahren hergestellt werden [vgl. J. Prakt. Chem. 315, 4, Seiten 791–793 (1973)].

Als inerte organische Verdünnungsmittel können alle für Phosgenierungen üblicherweise verwendeten Verdünnungsmittel eingesetzt werden. Beim Arbeiten in einem offenen System werden solche Verdünnungsmittel verwendet, deren Siedepunkt im Bereich der erfindungsgemäss anwendbaren Reaktionstemperatur liegt (150 bis 220 °C) oder deren Siedepunkt über dieser Reaktionstemperatur liegt. In Frage kommen beispielsweise gegebenenfalls halogenierte aliphatische und aromatische Kohlenwasserstoffe, wie Naphthalin oder durch Alkyl und/oder Halogen substituiertes Benzol und Naphthalin oder Tetrahydronaphthalin oder Benzonitril. Bevorzugt wird o-Dichlorbenzol verwendet.

Das erfindungsgemässe Verfahren wird im Temperaturbereich von 150 bis 220 °C durchgeführt. Bevorzugt arbeitet man bei 160 bis 190 °C, besonders bevorzugt bei 160 bis 180 °C. In Sonderfällen können die angegebenen Temperaturen auch unter- oder überschritten werden.

Das erfindungsgemässe Verfahren kann je nach apparativer Ausstattung bei erhöhtem Druck, z.B. im Autoklaven oder bei Normaldruck durchgeführt werden. Im allgemeinen arbeitet man bei Normaldruck.

Zur Durchführung des erfindungsgemässen Verfahrens werden zweckmässigerweise auf 1 Mol Ausgangsstoff der Formel II, mit $R^1$ = H oder die Gruppe der Formel IV, wenigstens 1 Mol Phosgen und auf 1 Mol Ausgangsstoff der Formel II, mit $R^1$ = Gruppe der Formel III, wenigstens 2 Mol Phosgen eingesetzt. Bei der Verfahrensdurchführung in offenen Systemen wird das Phosgen laufend in das Reaktionsgemisch eingeleitet und somit im allgemeinen ein Überschuss von Phosgen eingebracht.

Bei der Durchführung des erfindungsgemässen Verfahrens wird in offenen Systemen zweckmässigerweise der Ausgangsstoff der Formel II im Verdünnungsmittel gelöst oder suspendiert. Gegebenenfalls unter Rühren wird Phosgen eingeleitet und das Reaktionsgemisch auf die gewünschten Reaktionstemperaturen gebracht. Unter Erwärmen wird das Phosgen etwa 2 bis 5 Stunden eingeleitet. Die für die jeweilige Reaktion optimale Reaktionszeit kann in üblicher Weise leicht ermittelt werden. Anschliessend wird das Verdünnungsmittel destillativ entfernt und das erhaltene Rohprodukt durch Destillation im Vakuum gereinigt.

Entsprechend wird bei der Durchführung in geschlossenen Systemen gearbeitet. Hier wird die erforderliche Menge Phosgen unter Druck dem Reaktionsgemisch vor dem Erwärmen zugegeben.

Wie bereits oben dargelegt wurde, stellen die neuen Verbindungen der allgemeinen Formel I wertvolle Zwischenprodukte dar. In überraschender Weise sind die Endprodukte des erfindungsgemässen Verfahrens stabile Verbindungen, welche problemlos gelagert und gehandhabt werden können.

Die Verbindungen der allgemeinen Formel I lassen sich beispielsweise mit Aminen der allgemeinen Formel V

$$R^3\text{–}NH_2 \qquad (V)$$

in welcher

$R^3$ für einen gegebenenfalls substituierten aromatischen oder heteroaromatischen Rest steht,

zu substituierten Furazanen der allgemeinen Formel VI

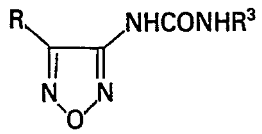

(VI)

in welcher R und $R^3$ die oben angegebenen Bedeutungen haben, umsetzen. Zur Herstellung der Verbindungen der allgemeinen Formel VI werden beispielsweise molare Mengen der Verbindungen der Formeln I und V in trockenem Toluol bei Temperaturen von ca. 80 °C umgesetzt und nach erfolgter Reaktion das Lösungsmittel destillativ entfernt. Die Verbindungen der Formel VI bleiben als feste Masse zurück.

Auf diese Weise können z. B. die folgenden Verbindungen der Formel VI erhalten werden:

| Verbindung | R | $R^3$ | Schmelzpunkt |
|---|---|---|---|
| A1 | 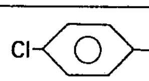 | 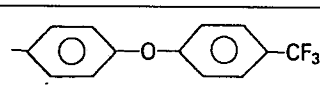 | 208 |
| A2 | 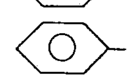 | 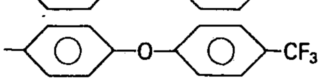 | 203 |

Die Verbindungen der Formel VI weisen hohe Wirksamkeiten gegen Insekten und Spinnmilben auf und können somit im Pflanzenschutz als Schädlingsbekämpfungsmittel verwendet werden.

Die akarizide Wirksamkeit der Verbindungen der Formel VI kann durch das folgende Beispiel demonstriert werden:

Entwicklungshemmtest mit Tetranychus urticae (Gemeine Spinnmilbe)

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Die Blätter der Bohnenpflanze (Phaseolus vulgaris), welche mit ca. 50 Eiern der Gemeinen Spinnmilbe belegt sind, taucht man in die entsprechend konzentrierte Wirkstoffzubereitung. Die Summe der abgetöteten Eier, Larven, Nymphen und Ruhestadien einer Generation, bezogen auf die Zahl eingesetzter Eier ergibt die Abtötung in %. Dabei bedeutet 100%, dass alle Tiere abgetötet wurden; 0% bedeutet, dass keine Tiere abgetötet wurden.

Bei diesem Test zeigen z. B. bei einer Wirkstoffkonzentration von 0,02% die Verbindungen A1 und A2 nach 12 Tagen eine Abtötung von 100%.

Die Verbindungen der Formel VI können in üblicher Weise durch Vermischen mit geeigneten Trägerstoffen (z. B. Gesteinsmehl oder Kieselsäure), Verdünnungsmittel (z. B. Dimethylformamid) und Emulgatoren (z. B. Dibutylnaphthalinsulfonat oder Dodecylbenzolsulfonsaures Calzium) in feste oder flüssige Schädlingsbekämpfungsmittel formuliert werden. Diese werden gegebenenfalls nach weiterer Verdünnung mit Wasser durch Stäuben oder Spritzen auf die zu behandelnden Pflanzen aufgebracht.

Das erfindungsgemässe Verfahren soll durch die folgenden Beispiele erläutert werden (alle %-Angaben beziehen sich, wo nicht anderes angegeben wird, auf Gewichts-%).

Beispiel 1

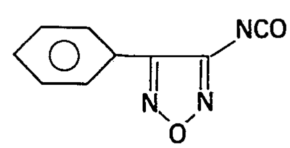

In einem 4-l-Reaktionsgefäss wird ein Gemisch von 293 g (1,82 mol) 3-Amino-4-phenyl-1,2,5-oxadiazol und 2 l destilliertes o-Dichlorbenzol vorgelegt und unter Rühren zügig auf 160 °C erhitzt. Dabei wird oberhalb von 60 °C Phosgen-Gas eingeleitet (ca. 100 g/h). Bei 160–165 °C wird 90 min lang weiter Phosgen eingeleitet, wobei aus der anfänglich klaren Lösung eine dicke, aber noch gut rührbare Suspension wird. Anschliessend wird das Gemisch zum Rückfluss erhitzt und 2,5 h

lang unter Rückfluss Phosgen eingeleitet, wobei eine klare Lösung entsteht. Das Gemisch wird durch Andestillieren bei Normaldruck von Phosgen befreit und dann im Wasserstrahlvakuum eingeengt. Das Konzentrat wird an der Ölpumpe destilliert und redestilliert.

Hierbei werden 278 g (82% der Theorie) 3-Isocyanato-4-phenyl-1,2,5-oxadiazol mit einem Kp 0,1 mbar von 85 °C und einem Schmelzpunkt von 53–54 °C erhalten.

Beispiel 2

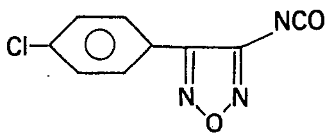

In einem 4-l-Reaktionsgefäss wird ein Gemisch von 300 g (1,53 mol) 3-Amino-4-(4'-chlorphenyl)-1,2,5-oxadiazol und 2,6 l destilliertem o-Dichlorbenzol vorgelegt und unter Rühren zügig auf 175 °C (Rückfluss) erhitzt. Dabei wird oberhalb 60 °C Phosgen-Gas eingeleitet (ca. 100 g/h). Bei etwa 80 °C wird das Gemisch klar und bleibt auch während der gesamten Umsetzung klar. Nach 5 h Phosgeneinleitung unter Rückfluss wird das Gemisch durch Andestillieren bei Normaldruck von Phosgen befreit und dann im Wasserstrahlvakuum eingeengt. Das Konzentrat wird im Ölpumpenvakuum destilliert.

Auf diese Weise werden 327 g (96% der Theorie) 3-Isocyanato-4-(4'-chlorphenyl)-1,2,5-oxadiazol mit Kp 0,05 mbar von 87–88 °C und einem Schmelzpunkt von 66° erhalten.

Beispiele 3–9

Nach der in den Beispielen 1 und 2 angegebenen Verfahrensweise werden die folgenden Verbindungen der Formel VII erhalten:

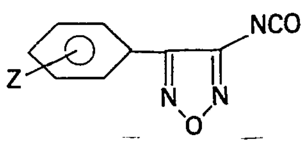

(VII)

| Beispiel Nr. | Z | Schmelzpunkt (°C) | Siedepunkt $K_2$mbar (°C) |
|---|---|---|---|
| 3 | 2-Cl | | 101–103 ($n_D^{20}$=1,5646)* |
| 4 | 2-Cl, 4-Cl | 67 | 125 |
| 5 | 4-F | 80 | 83 |
| 6 | 4-Br | 82 | 125 |
| 7 | 4-$CH_3$ | 52 | 105–106 |
| 8 | 3-Cl, 4-Cl | 40 | 132 |
| 9 | 4-$SCF_3$ | | 116 ($n_D^{20}$=1,5378)* |

* Brechungsindex

Beispiel 10

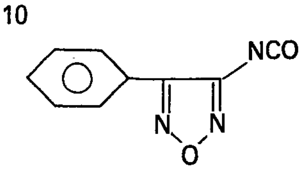

In einem 4-l-Reaktionsgefäss wird eine Suspension von 140 g (0,4 mol) N,N'-Bis-(4-phenyl-1,2,5-oxadiazol-3-yl)-harnstoff und 2 l destilliertes o-Dichlorbenzol vorgelegt und unter Rühren zügig auf 160 °C, dann im Verlauf von 1 h auf 175 °C (Rückfluss) erwärmt. Dabei wird oberhalb von 100 °C Phosgen-Gas eingeleitet (ca. 100 g/h). Es wird dann noch eine weitere Stunde unter Rückfluss Phosgen eingeleitet, wobei das Gemisch langsam in eine klare Lösung übergeht. Anschliessend wird das Gemisch durch Andestillieren bei Normaldruck von Phosgen befreit und dann im Wasserstrahlvakuum eingeengt. Das Konzentrat wird schliesslich im Ölpumpenvakuum destilliert. Nach einem Vorlauf (15,8 g Dichlorbenzol, 7,2 g 3-Isocyanato-4-phenyl-1,2,5-oxadiazol) werden als Hauptfraktion beim Kp 0,1 mbar 85 °C 118 g 3-Isocyanato-4-phenyl-1,2,5-oxadiazol als farbloses Produkt erhalten.

Gesamtausbeute: 125 g ≙ 83% der Theorie.

Schmelzpunkt des Produktes: 53–54 °C.

Beispiel 10a (Ausgangsstoff für Beispiel 10)

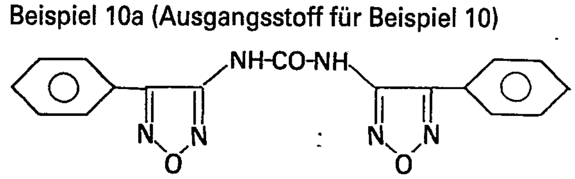

In einem 4-l-Reaktionsgefäss wird ein Gemisch von 161 g (1,0 mol) 3-Amino-4-phenyl-1,2,5-oxadiazol und 2 l destilliertes Chlorbenzol vorgelegt und unter Rühren erwärmt. In das Gemisch wird Phosgen-Gas eingeleitet (ca. 100 g/h). Oberhalb von 60 °C fällt ein farbloser, feinteiliger Niederschlag aus. Nach 2 h wird eine Temperatur von 130 °C (Rückfluss) erreicht. Es wird noch 30 min lang Phosgen unter Rückfluss eingeleitet. Die entstandene, gut rührbare Suspension wird durch Andestillieren von Phosgen befreit, dann abgekühlt und filtriert: N,N'-Bis-(4-phenyl-1,2,5-oxadiazol-3-yl)-harnstoff in Form von 147 g fast farblosen Kristallen vom Schmelzpunkt 280 °C wird erhalten. Durch Einengen des Filtrates werden weitere 21 g des Produktes in Form von bräunlichen Kristallen vom Schmelzpunkt 280 °C erhalten.

Das Produkt kann durch Umkristallisieren aus Chlorbenzol gereinigt werden. Die hierbei anfallenden farblosen Kristalle haben einen Schmelzpunkt von 280–281 °C.

Die übrigen als Ausgangsstoffe verwendbaren Harnstoffe können entsprechend hergestellt werden.

Beispiel 11

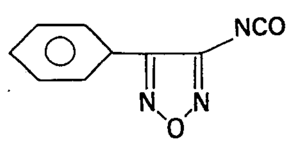

Zu 400 ml o-Dichlorbenzol werden 52 g (0,2 mol) N-n-Butyl-N'-(4-phenyl-1,2,5-oxadiazol-3-yl)-harnstoff (Schmelzpunkt 155 °C) gegeben und zum Rückfluss erhitzt. Dabei wird mit einer Geschwindigkeit von ca. 100 g/h Phosgen eingeleitet, bis der Harnstoff vollkommen umgesetzt ist (klare Lösung). Nach der Entfernung des überschüssigen Phosgens durch Unterdruck wird das Lösungsmittel zusammen mit dem n-Butylisocyanat abdestilliert und der Rückstand im Vakuum destilliert. Bei einem Kp 2 mbar 82–84 °C wurden 29 g (77,4% der Theorie) 3-Isocyanato-4-phenyl-1,2,5-oxadiazol erhalten (Schmelzpunkt 53–54 °C).

Beispiel 12

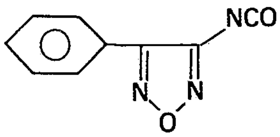

56 g (0,21 mol) N-Phenyl-N'-(4-phenyl-1,2,5-oxadiazol-3-yl)-harnstoff (Schmelzpunkt 213 °C) werden in 500 ml o-Dichlorbenzol zum Rückfluss erhitzt. In das Gemisch wird Phosgen in einer Menge von 100 g/h eingeleitet, bis der Harnstoff vollständig umgesetzt ist (klare Lösung). Das überschüssige Phosgen wird durch leichten Unterdruck entfernt und das Lösungsmittel sowie das entstandene Phenylisocyanat abdestilliert. Beim anschliessenden Destillieren unter Vakuum werden 24 g (64,2% der Theorie) 3-Isocyanato-4-phenyl-1,2,5-oxadiazol erhalten (Schmelzpunkt 53–54 °C).

Die Herstellung der insektizid und akarizid wirkenden Verbindungen der Formel VI kann anhand des folgenden Beispiels erläutert werden:

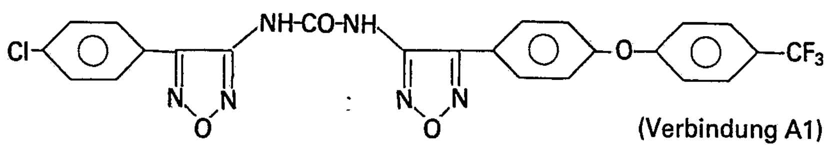

(Verbindung A1)

---

2,53 g (0,01 mol) 4-(4-Trifluormethylphenoxy)-anilin werden bei 60 °C in 60 ml trockenem Toluol gelöst. Nach Zugabe von 2,2 g (0,01 mol) 3-Isocyanato-4-(4-chlorphenyl)-1,2,5-oxadiazol in 10 ml trockenem Toluol wird 30 min bei 80 °C gerührt und anschliessend die Hauptmenge des Lösungsmittels abdestilliert. Nach Abtrennung des Rückstands wird getrocknet.

Man erhält 3,6 g (76% der Theorie) N-[4-(4-Trifluormethylphenoxy)-phenyl]-N'-[4-(4-chlorphenyl)-1,2,5-oxadiazol-3-yl)]-harnstoff vom Schmelzpunkt 208 °C.

Die übrigen Verbindungen der Formel VI können gemäss diesem Beispiel hergestellt werden.

**Patentansprüche**

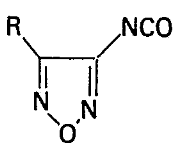

(I)

1. Verfahren zur Herstellung von 3-Isocyanato-1,2,5-oxadiazolen der allgemeinen Formel I

in welcher

R für einen gegebenenfalls substituierten aromatischen Rest, welcher unter den erfindungsgemässen Verfahrensbedingungen mit Phosgen nicht reagiert, steht,

dadurch gekennzeichnet, dass man Verbindungen der allgemeinen Formel II

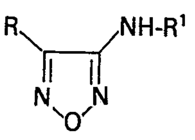

(II)

in welcher

R die oben angegebene Bedeutung hat und

$R^1$ für Wasserstoff oder für die Gruppen der allgemeinen Formeln III oder IV

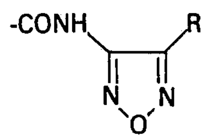

(III)

$-CONHR^2$ (IV)

steht, in welchen

R die oben angegebene Bedeutung hat und

$R^2$ einen gegebenenfalls substituierten aliphatischen oder aromatischen Rest bedeutet,

mit Phosgen bei Temperaturen zwischen 150 und 220 °C in Gegenwart eines organischen Verdünnungsmittels umsetzt und das erhaltene Isocyanat der allgemeinen Formel I nach den üblichen Methoden isoliert und gegebenenfalls reinigt.

2. Verfahren gemäss Anspruch 1, wobei R für Phenyl, welches durch Halogen, Nitro, Cyano, $C_1$–$C_4$-Alkyl, $C_1$–$C_4$-Alkoxy, $C_1$–$C_4$-Alkylthio, Halogen-$C_1$–$C_4$-Alkyl, Halogen-$C_1$–$C_4$-Alkoxy, Halogen-$C_1$–$C_4$-Alkylthio, Halogen-$C_1$–$C_4$-Alkylcarbonyl, $C_1$–$C_4$-Alkylcarbonyl, Halogen-$C_1$–$C_4$-Alkoxycarbonyl, $C_1$–$C_4$-Alkoxycarbonyl, $C_1$–$C_4$-Alkoxycarbonyl-$C_1$–$C_4$-Alkyl und/oder $C_1$–$C_4$-Alkoxycarbonyl-$C_1$–$C_4$-Alkylthio substituiert sein kann, steht.

3. Verfahren gemäss Anspruch 1, wobei R für Phenyl, welches substituiert sein kann durch Fluor, Chlor, Brom, Cyano, Nitro, Methyl, Ethyl, Methoxy, Ethoxy, Methylthio, Trifluormethyl, Trifluormethoxy, Trifluormethylthio, Methylcarbonyl, Methoxycarbonyl, Ethoxycarbonyl, n-Butoxycarbonyl, tert.-Butoxycarbonyl, Methoxycarbonylmethyl, Ethoxycarbonylmethyl, Methoxycarbonylmethylthio und/oder Ethoxycarbonylmethylthio steht.

4. Verfahren gemäss Anspruch 1, wobei R für Phenyl, welches durch Fluor, Chlor und/oder Brom substituiert sein kann, steht.

5. Verfahren gemäss den Ansprüchen 1 bis 4, wobei $R^1$ für Wasserstoff, für die Gruppe der allgemeinen Formel III, in welcher R die in den Ansprüchen 1 bis 4 angegebene Bedeutung hat oder für die Gruppe der allgemeinen Formel IV, in welcher $R^2$ $C_1$–$C_6$-Alkyl oder Phenyl bedeutet, steht.

6. Verfahren gemäss den Ansprüchen 1 bis 4, wobei $R^1$ für Wasserstoff, für die Gruppe der allgemeinen Formel III, in welcher R die in den Ansprüchen 2 bis 4 angegebene Bedeutung hat oder für die Gruppe der allgemeinen Formel IV, in welcher $R^2$ $C_1$–$C_4$-Alkyl oder Phenyl bedeutet, steht.

7. Verfahren gemäss den Ansprüchen 1 bis 4, wobei $R^1$ für Wasserstoff oder für die Gruppe der allgemeinen Formel III, in welcher R Phenyl oder Chlorphenyl (vorzugsweise 4-Chlorphenyl) bedeutet, steht.

8. Verfahren gemäss den Ansprüchen 1 bis 4, wobei $R^1$ für Wasserstoff steht.

9. Verfahren gemäss den Ansprüchen 1 bis 8, dadurch gekennzeichnet, dass man die Umsetzung bei Temperaturen zwischen 160 und 190 °C vornimmt.

10. Verfahren gemäss den Ansprüchen 1 bis 8, dadurch gekennzeichnet, dass man die Umsetzung bei Temperaturen zwischen 160 und 180 °C vornimmt.

**Claims**

1. Process for the preparation of 3-isocyanato-1,2,5-oxadiazoles of the general formula I

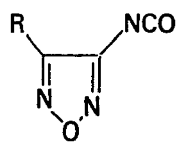

(I)

in which

R represents an optionally substituted aromatic radical which does not react with phosgene under the process conditions according to the invention, characterized in that compounds of the general formula II

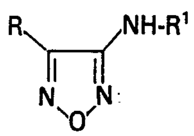

(II)

in which

R has the meaning given above and $R^1$ represents hydrogen or the groups of the general formulae III or IV

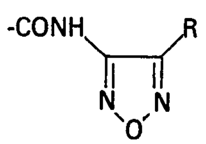

(III)

$-CONHR^2$ (IV)

in which

R has the meaning given above and

$R^2$ denotes an optionally substituted aliphatic or aromatic radical,

are reacted with phosgene at temperatures between 150 and 220 °C in the presence of an organic diluent, and the resulting isocyanate of the general formula I is isolated by the customary methods and, if required, purified.

2. Process according to claim 1, wherein R represents phenyl which can be substituted by halogen, nitro, cyano, $C_1$–$C_4$-alkyl, $C_1$–$C_4$-alkoxy, $C_1$–$C_4$-alkylthio, halogeno-$C_1$–$C_4$-alkyl, halogeno-$C_1$–$C_4$-alkoxy, halogeno-$C_1$–$C_4$-alkylthio, halogeno-$C_1$–$C_4$-alkylcarbonyl, $C_1$–$C_4$-alkylcarbonyl, halogeno-$C_1$–$C_4$-alkoxycarbonyl, $C_1$–$C_4$-alkoxycarbonyl, $C_1$–$C_4$-alkoxycarbonyl-$C_1$–$C_2$-alkyl and/or $C_1$–$C_4$-alkoxycarbonyl-$C_1$–$C_4$-alkylthio.

3. Process according to claim 1, wherein R represents phenyl which can be substituted by fluorine, chlorine, bromine, cyano, nitro, methyl, ethyl, methoxy, ethoxy, methylthio, trifluoromethyl, trifluoromethoxy, trifluoromethylthio, methylcarbonyl, methoxycarbonyl, ethoxycarbonyl, n-butoxycarbonyl, tert.-butoxycarbonyl, methoxycarbonylmethyl, ethoxycarbonylmethyl, methoxycarbonylmethylthio and/or ethoxycarbonylmethylthio.

4. Process according to claim 1, wherein R represents phenyl which can be substituted by fluorine, chlorine and/or bromine.

5. Process according to claims 1 to 4, wherein $R^1$ represents hydrogen, the group of the general formula III in which R has the meaning given in claims 1 to 4, or the group of the general formula IV in which $R^2$ denotes $C_1$–$C_6$-alkyl or phenyl.

6. Process according to claims 1 to 4, wherein $R^1$ represents hydrogen, the group of the general formula III in which R has the meaning given in claims 2 to 4, or the group of the general formula IV in which $R^2$ denotes $C_1$–$C_4$-alkyl or phenyl.

7. Process according to claims 1 to 4, wherein $R^1$ represents hydrogen or the group of the general formula III in which R denotes phenyl or chlorophenyl (preferably 4-chlorophenyl).

8. Process according to claims 1 to 4, wherein $R^1$ represents hydrogen.

9. Process according to claims 1 to 8, characterized in that the reaction is carried out at temperatures between 160 and 190 °C.

10. Process according to claims 1 to 8, characterized in that the reaction is carried out at temperatures between 160 and 180 °C.

**Revendications**

1. Procédé de préparation de 3-isocyanato-1,2,5-oxadiazoles de formule générale I

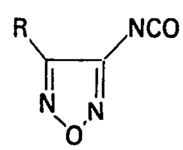

(I)

dans laquelle

R représente un radical aromatique éventuellement substitué qui, dans les conditions opératoires selon l'invention, ne réagit pas avec le phosgène,

caractérisé en ce que l'on fait réagir des composés de formule générale II

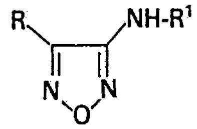

(II)

dans laquelle

R a les significations indiquées ci-dessus, et

$R^1$ représente l'hydrogène ou les groupes de formules générales III ou IV

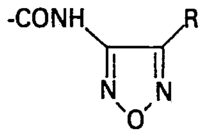

(III)

$-CONHR^2$ (IV)

dans lesquelles

R a les significations indiquées ci-dessus, et

$R^2$ représente un radical aliphatique ou aromatique éventuellement substitué, avec le phosgène, à des températures de 150 à 220 °C, en présence d'un diluant organique, on isole l'isocyanate obtenu de formule générale I par des techniques usuelles et le cas échéant on le modifie.

2. Procédé selon la revendication 1, dans lequel R représente un groupe méthyle qui peut être substitué par des halogènes, des groupes nitro, cyano, alkyle en $C_1$–$C_4$, alcoxy en $C_1$–$C_4$, alkylthio en $C_1$–$C_4$, halogénoalkyle en $C_1$–$C_4$, halogénoalcoxy en $C_1$–$C_4$, halogénoalkylthio en $C_1$–$C_4$, halogéno-(alkyle en $C_1$–$C_4$)-carbonyle, (alkyle en $C_1$–$C_4$)-carbonyle, halogéno-(alcoxy en $C_1$–$C_4$)-carbonyle, (alcoxy en $C_1$–$C_4$)-carbonyle, (alcoxy en $C_1$–$C_4$)-carbonylalkyle en $C_1$–$C_4$ et/ou (alcoxy en $C_1$–$C_4$)-carbonyl-alkylthio en $C_1$–$C_4$.

3. Procédé selon la revendication 1, dans lequel R représente un groupe phényle qui peut être substitué par le fluor, le chlore, le brome, des groupes cyano, nitro, méthyle, éthyle, méthoxy, éthoxy, méthylthio, trifluorométhyle, trifluorométhoxy, trifluorométhylthio, méthylcarbonyle, méthoxycarbonyle, éthoxycarbonyle, n-butoxycarbonyle, tert-butoxycarbonyle, méthoxycarbonylméthyle, éthoxycarbonylméthyle, méthoxycarbonylméthyltio et/ou éthoxycarbonylméthylthio.

4. Procédé selon la revendication 1, dans lequel R représente un groupe phényle qui peut être substitué par le fluor, le chlore et/ou le brome.

5. Procédé selon les revendications 1 à 4, dans lequel $R^1$ représente l'hydrogène, le groupe de formule générale III dans laquelle R a les significations indiquées dans les revendications 1 à 4 ou le groupe de formule générale IV dans laquelle $R^2$ représente un groupe alkyle en $C_1$–$C_6$ ou phényle.

6. Procédé selon les revendications 1 à 4, dans lequel $R^1$ représente l'hydrogène, le groupe de formule générale III dans laquelle R a les significations indiquées dans les revendications 2 à 4 ou le groupe de formule générale IV dans laquelle $R^2$ représente un groupe alkyle en $C_1$–$C_4$- ou phényle.

7. Procédé selon les revendications 1 à 4, dans lequel $R^1$ représente l'hydrogène ou le groupe de formule générale III dans laquelle R représente un groupe phényle ou chlorophényle (de préférence 4-chlorophényle).

8. Procédé selon les revendications 1 à 4, dans lequel $R^1$ représente l'hydrogène.

9. Procédé selon les revendications 1 à 8, caractérisé en ce que l'on effectue la réaction à des températures de 160 à 190 °C.

10. Procédé selon les revendications 1 à 8, caractérisé en ce que l'on effectue la réaction à des températures de 160 à 180 °C.